# EUROPEAN PATENT SPECIFICATION

(11) **EP 2 600 806 B1**
(45) Date of publication and mention of the grant of the patent: **30.10.2019**
(21) Application number: 11815168.7
(22) Date of filing: 02.08.2011
(51) Int. Cl.: A61F 5/01, A61F 5/058, A61F 5/32

(54) **FLEXIBLE ANATOMICAL SUPPORT**
FLEXIBLE ANATOMISCHE STÜTZE
SUPPORT ANATOMIQUE FLEXIBLE

(30) Priority: 01.08.2011 US 201113195767; 02.08.2010 US 369921 P
(43) Date of publication of application: 12.06.2013
(73) Proprietor: Ovation Medical, Thousand Oaks, CA 91362 (US)
(72) Inventor: GRIM, Tracy, E., Thousand Oaks CA 91362 (US); IGLESIAS, Joseph, Michael, Thousand Oaks CA 91362 (US); SCOTT, Eric, Gerald, Thousand Oaks CA 91362 (US); FRANCO, Justina-Delia, A., Thousand Oaks CA 91362 (US)
(74) Representative: Dolleymores
(86) International application number: PCT/US2011/046226
(87) International publication number: WO 2012/018785

(56) References cited:
- EP-A1- 2 453 849
- WO-A1-02/17827
- WO-A1-92/19196
- WO-A1-95/31950
- WO-A2-2006/084220
- WO-A2-2007/050703
- US-A- 4 193 135
- US-A- 4 840 168
- US-A- 4 893 616
- US-A- 5 014 689
- US-A- 5 445 602
- US-A- 5 971 946
- US-A- 5 980 476
- US-A1- 2005 121 562
- US-A1- 2005 273 030
- US-A1- 2009 012 438
- US-A1- 2010 094 189
- US-B1- 6 496 984

## Description

### BACKGROUND

### Field

The present disclosure relates to flexible anatomical wrist and thumb braces that include a cushioning material to reduce irritation and fitment discomfort to a user.

### Background

A flexible support, such as a wrist brace is used to provide compression and support for wrist, hand and forearm for management of post fracture or soft tissue injuries and other conditions. The thumb protrudes through a thumbhole to allow function of the thumb. The hand and fingers extend from the distal portion of the brace and allow for the most part 90° flexion at the metacarpal phalangeal (MP) Joint. Typically, a liner fabric covers the interior of the brace to provide some comfort where skin contact is made. For the most part, there is restricted motion between the brace and most of the hand. However, thumb motion may lead to irritation of the skin where the thumb crosses over the web space of the wrist brace during pitching motion, and most especially at stitching (binding) around the edge of the thumbhole where layers of material are sewn together. It may also have friction at MP joint of hand at the palmar crease. It is desirable to amend the structure of the brace in this and other locations to provide added cushioning and reduce skin irritation from friction due to hand/finger motion during use of hand while wearing. Particularly when the hand is tender, swollen from injuries, and/or pathological conditions. Preferably, the concepts for amending the structure should be applicable to other flexible supports. International Patent Application, publication number WO 92/19196 A1, discloses a known wrist support having a thumb hole, WO2006/084220A1 discloses a wrist support with a strap passing between the thumb and forefinger and US Patent Number US 4,840,168 discloses a support for a thumb.

A wrist brace in accordance with the preamble of claim 1 is known from WO2007/050703 A2.

### SUMMARY

In accordance with the present invention there is provided a wrist brace as recited in claim 1.

The present invention will become readily apparent to those skilled in the art from the following detailed description, of exemplary configurations of wrist braces in accordance with the invention. As will be realized, the present invention includes other and different examples and its several details are capable of modification in various respects, all without departing from the scope of the present invention. Accordingly, the drawings and the detailed description are to be regarded as illustrative in nature and not as restrictive.

The invention, however, is defined by the scope of the appended claims.

### BRIEF DESCRIPTION OF THE DRAWINGS

FIG. 1A shows a perspective view illustrating an example of an insert attached to a brace.
FIG. 1B shows another perspective view of FIG. 1A.
FIG. 2A shows a perspective view illustrating an example of a donut-shaped insert in accordance with the disclosure.
FIG. 2B shows a perspective side-view of the donut-shaped insert of FIG. 2A.
FIG. 2C shows another perspective view of a brace including the donut-shaped insert of FIG. 2A.
FIG. 3 shows a perspective view illustrating an example of a brace including a saddle insert.
FIG. 4A shows a perspective view illustrating an example of a brace including a long saddle insert.
FIG. 4B shows a detail cross-section view of the insert of FIG. 4A.
FIG. 5 shows a cut away perspective view illustrating an example of a donut insert including a hollow space.
FIG. 6 shows a section view illustrating an example of an insert including compressible cushion material bonded to a portion of stretch-resistant material.
FIG. 7 shows a perspective view illustrating an example of a pre-cut insert including compressible cushion material and an adhesive layer.
FIG. 8 shows a perspective view illustrating an example of an insert attached to a portion of the thumbhole of a brace, the insert containing a hollow portion.
FIG. 9 shows a perspective view illustrating an example an insert attached to a portion of the thumbhole of a brace, the insert containing a pillow of foam beads in a hollow portion.
FIG. 10 shows a perspective view illustrating an example an insert attached to a portion of the thumbhole of a brace, the insert containing a recessed region for receiving an inlay.
FIG. 11A shows a side view illustrating an example of a brace web space with a cut in the thumbhole for adding an insert.
FIG. 11B shows a tube insert attached to a portion of the thumbhole of a brace in accordance with the disclosure.
FIG. 11C shows a perspective view illustrating an example of brace web space with a tube insert over a reinforcement strip.
FIG. 12 shows a side view illustrating an example of an insert attached to an orthotic brace with a thumb stabilizer in accordance with the disclosure.
FIG. 13A shows a side view illustrating an example of a foam tape insert attached to a web region of an orthotic wrist brace.
FIG. 13B is a detailed view FIG. 13A showing the foam tape attached to the web region of the orthotic wrist brace.
FIG. 14A shows a spacer pad insert attached to an interior surface of a brace in accordance with the disclosure.
FIG. 14B shows a cross-section view illustrating an example of a spacer pad.
FIG. 15 shows a cross-section view illustrating an example of a long spacer pad located along the palmar stay region of an orthotic wrist brace.
FIG. 16 illustrates an example of a method for making an orthotic wrist brace, which wrist brace is in accordance with the present invention, the wrist brace including a molded insert, a web area component and a main brace body part.

### DETAILED DESCRIPTION

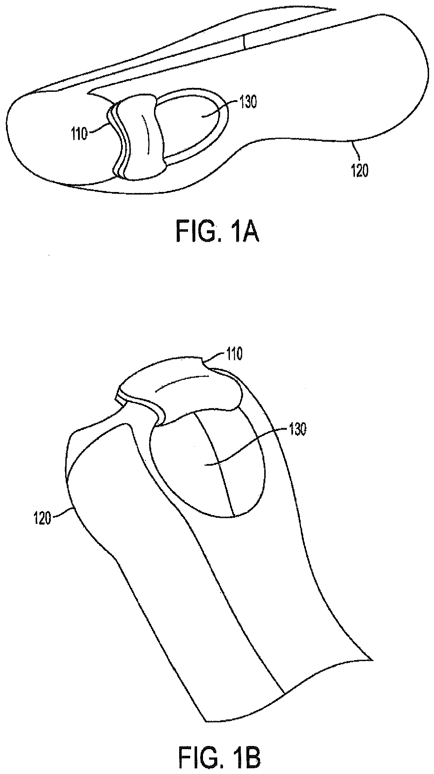

Various inserts for use with a wrist brace are disclosed. For the purposes of this disclosure, an "insert" is a material for the flexible support to reduce irritation and fitment discomfort to the user. The insert may be integrally formed with the flexible support or manufactured separately for attachment to a flexible support. FIG. 1A shows an example of an insert 110 attached to a brace 120. Insert 110 includes a material that is softer and more flexible than the materials from which the brace 120 is made, which includes support stays and edge binding. The insert 110 is applied to a portion of the brace forming a thumbhole 130 to provide cushioning in a portion of the hand web region between the thumb and the index finger, where irritation is a known issue due to rubbing between the brace material and the skin.

FIG. 1B shows a detail view illustrating an example of the insert 110. The insert 110 may be shaped to a portion of the brace 120 to which it is applied, and may be attached to the brace using a variety of attachment means, including stitching, adhesives, such as glues, fabric hook and loop fasteners, such as Velcro™, and the like.

The insert 110 may comprise one or more layers of one or more different materials. In an embodiment, the insert 110 may be an integral component of the brace 120, and be attached to the brace in the thumb and web space areas of the hand. In another embodiment, the insert may be supplied independent of the brace and attached to the brace in the thumb and web space areas of the hand using any of a variety of adhesive and/or bonding methods.

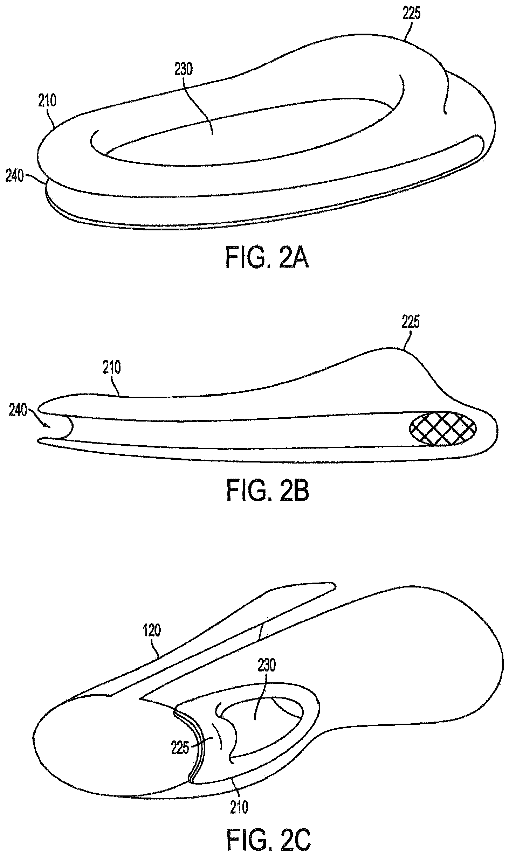

The insert 110 may have various shapes, depending on the customized need or preference of the user. FIG. 2A shows a perspective view illustrating an example of a "donut" insert 210. FIG. 2B shows a side-view illustrating an example of the donut insert 210. The donut insert 210 is approximately ring shaped to fit into the thumbhole 130, and has a corresponding insert thumbhole 230. The donut insert 210 is preferably molded and may have a contoured shape, such as a ridge 225, to provide added cushion at the base of the thumb adjacent to the web of the hand. The donut insert 210 may further have a groove 240 to facilitate insertion and alignment with an interior edge of the thumbhole 130. If the brace 120 includes a bridge across the hand web from the front to back of the hand, that is, if the thumbhole 130 is a complete circle around the thumb, the groove 240 may completely encircle the donut insert 210 to contact the entire thumbhole 130. If the thumbhole 130 is only a partial circle and open in the web area, the groove 240 may be molded to not fully encircle the donut insert 210, but provide extra cushioning material in the web area.

FIG. 2C shows a perspective view illustrating an example of the brace 120 with the donut insert 210 attached to the wrist brace 120.

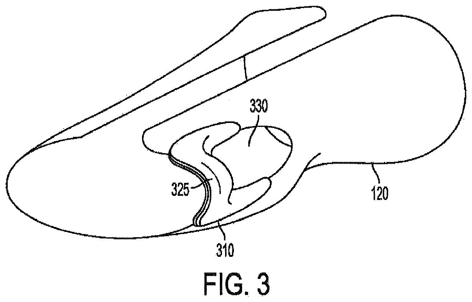

FIG. 3 shows an example of the wrist brace 120 with a "saddle" insert 310. Saddle insert 310 spans the hand web space from the front of the hand to the back, between the thumb and the opposing fingers, but does not completely encircle the thumb, and attaches to only a portion of the thumbhole. Saddle insert 310 may include one or more contours, such as ridge 325, to provide additional cushioning and comfort.

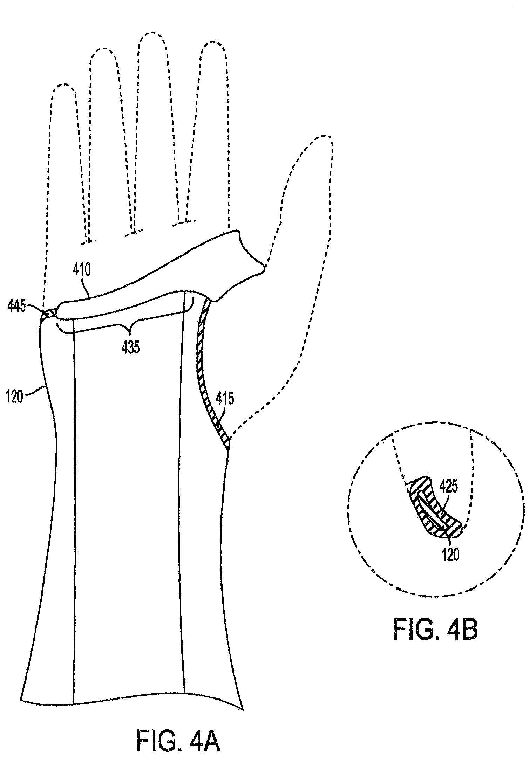

FIG. 4A shows an example of the wrist brace 120 with a "long saddle" insert 410, which is a variation of the saddle insert 310 including a cushioned palmar extension 435 as an integral part of the long saddle insert 410. Palmar extension 435 provides padding comfort and protection across the palmar crease region of the hand where binding stitch-work 445 can irritate the skin, such as from rubbing.

FIG. 4B is a cross-section detail view illustrating an example of how the long saddle insert 410 may be contoured to conform to the curve of the web at the base of the thumb. The long saddle insert 410, like all other inserts (e.g., 110, 210, 310 and variations) may include contoured curved surfaces 425 to follow the line of the web space, and enable ease of thumb flexion.

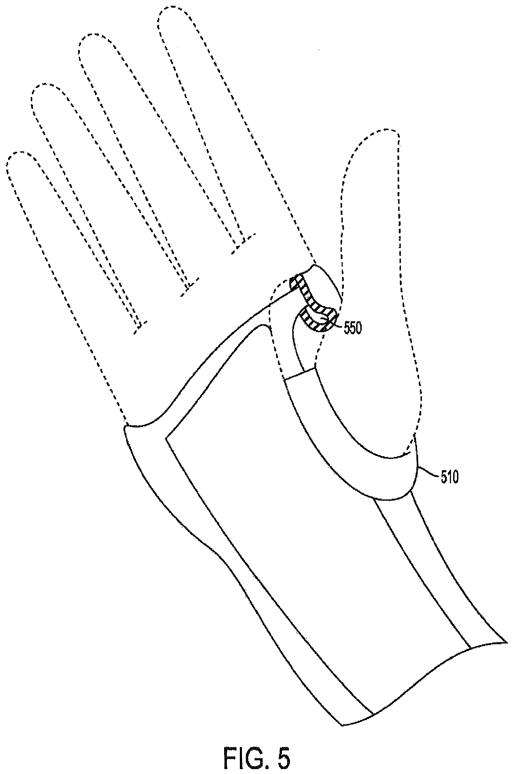

FIG. 5 shows a cut-away view illustrating an example of a hollow donut insert 510 attached to the wrist brace 120. The hollow donut insert 510 is a variant of the donut insert 210 in that a hollow pocket space 550 is provided which can be filled with other materials to tailor the fit and firmness of the insert, as described below. The hollow pocket space 550 may be incorporated in any insert previously described and in any variants that may arise for custom requirements.

The insert can be made from a variety of materials, individually or in combination, where the key features include a combination of softness of feel for skin comfort, compression resistance for support, and breathability for air-flow and temperature comfort. Exemplary materials include foam compositions (e.g., open cell or closed cell), knit, woven/non-woven fabrics and felts. The fabric, in one embodiment, may be laminated to one or both sides of the foam to form a multilayered material, from which the insert is fabricated. Preferentially, the foam would have a soft feel to the touch and having a low coefficient of friction (COF) while providing an adequate amount of compression resistance for padding with support at the edges of the brace and in the web space and palmar crease area of the hand. In another embodiment, the insert may be a non-laminate, consisting of a foam material with no additional materials bonded to the foam, while providing a low coefficient of friction (COF) with an adequate amount of compression resistance for padding with support at the edges of the brace and in the web space and palmar crease area of the hand.

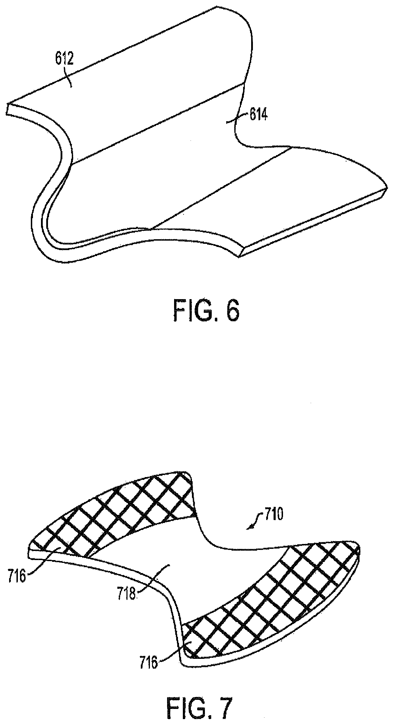

Lamination may be advantageous for various reasons. For example, when the foam is soft and stretchable, and may be subject to tearing under excessive use or strain, a lamination to a relatively stronger fabric with limited or no stretch ability can provide the added strength and stability to preserve an adequate service life of the insert. For example, FIG. 6 shows an example of a section or piece of insert 610 which include soft, stretchable foam 612 (or equivalent material) with at least a portion of one surface of the foam 612 laminated to a relatively non-stretch material 614 for strength. Insert 612 may be a pre-cut piece for later addition to a wrist brace 120, or may be integrally molded or shaped and manufactured with the brace 120.

FIG. 7 shows an example of a pre-cut insert 710, which includes foam, and further includes adhesive layers 716 laminated to foam 718 for attaching the pre-cut insert 710 to any wrist brace 120. Two separate areas of laminated adhesive are shown in FIG. 7 as one exemplary set of locations, but other configurations are possible. The adhesive layers 716 may attach directly to the brace 120 or, for example, by wrapping around the portion of the loop of the thumbhole 130 that crosses over the web area, and attach to each other. Various adhesive means may be used, including adhesive tapes, fabric hook and loop fasteners, such as Velcro™, heat welding, mechanical fasteners, glues, epoxies, hot melt glues, contact cement, heat and/or catalyst activated adhesive films, or other suitable securing means, and attachment may occur at various locations of the brace, according to the user's requirements. Mechanical fasteners may include sewing, hooks, buttons, etc., in which case material lamination may not be part of the adhesive system of attachment. Lamination of adhesives may be combined with lamination of other materials for differing mechanical purposes, and in the same or different portions of the insert.

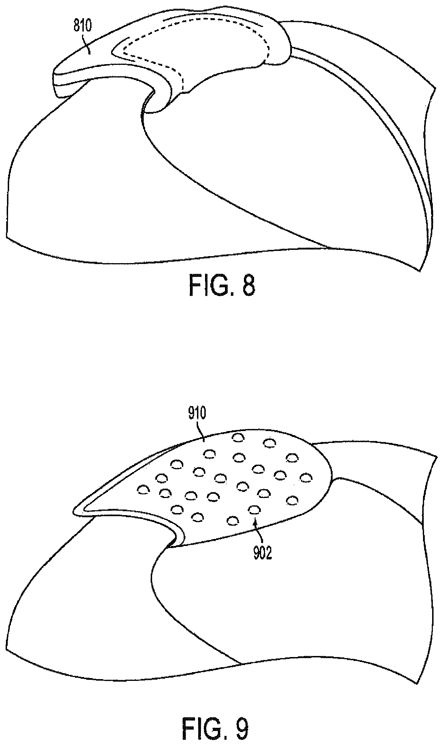

FIG. 8 shows an example of a pocket insert 810, which is similar in function to the hollow donut insert 510. The pocket insert 810, as shown in one exemplary embodiment, as attached to the web portion of the thumbhole 130, includes a hollow space for incorporating a filler material for various purposes. For example, the filler may be a clay-like or wax-like material that molds to the shape of the hand and the brace at the contact surfaces to distribute contact force. Other filler materials may include foam beads, small solid beads (e.g., like a bean bag), and other materials with specific compressive and/or shaping properties, as required. Adhesive means, as discussed above, may be combined in the pocket insert 810 for attachment to the brace as well as to stabilize the location of the filler material within the pocket insert.

FIG. 9 shows an example of a "pillow" insert 910 attached to a portion of the loop of the thumbhole 130 of the brace 120, where the insert contains a pillow of foam beads in the hollow portion.

The exterior surface of the foam that comes in contact with the skin may further include a finish and texture that improves the contact "feel" and comfort by minimizing the coefficient of friction (COF) and may also enhance air flow to enable moisture release and reduce uncomfortable heat buildup at locations of skin contact with the brace 120. For example, the pillow insert 910 of FIG. 9 shows a textured surface 902 to enhance skin comfort. The textured surface 902 can be combined with any shaped insert.

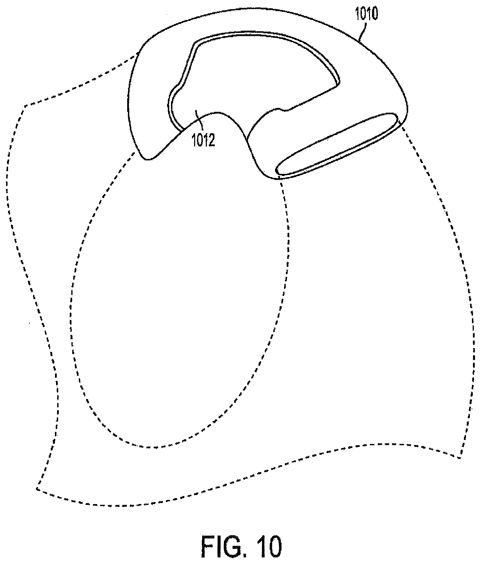

FIG. 10 shows an example of an insert 1010 attached to a portion of the thumbhole 130 of a brace, the insert 1010 containing a recessed region 1012 for receiving an inlay of material having defined material characteristics depending on a user's requirements. The inlays may facilitate additional comfort or other functions. The inlays may be configured to cover selected areas where the skin makes contact with the brace insert. The inserts may comprise various materials such as, for example, foams having different density and/or compressibility, such as, for example, memory foam, slow recovery foam, cold-forming material that conformably shapes due to pressure and time to customize the shape of the insert to the user's anatomy for improved comfort and any material and/or surface texture that provides a defined feel.

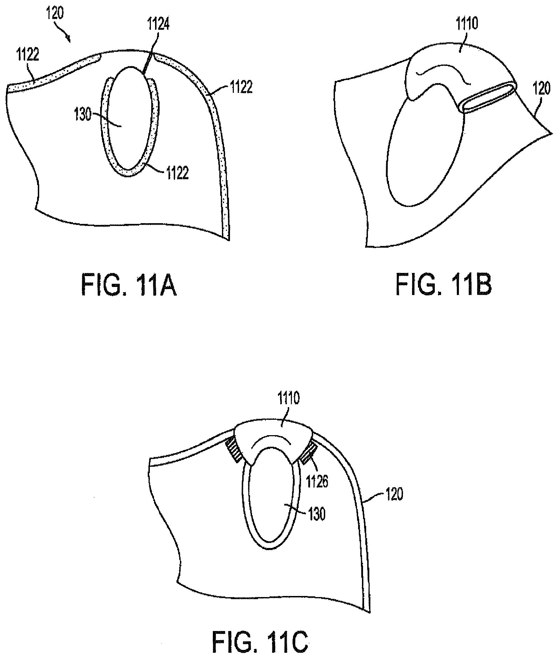

FIG. 11A shows an example of the web space of a brace with a cut in the thumbhole area for adding an insert to the brace. Stitch-work binding 1122 may extend along all or some edges of the brace 120. In the web space of the thumbhole 130 a cut 1124 can be made. FIG. 11B shows an example of a hollow "tube web insert" 1110 that may be inserted via the cut 1124 over the web space area of the brace 120. The tube web insert 1110 may be slid past the cut 1124 in order to sew or refasten the cut 1124. The tube web insert 1110 may be molded, extruded, or fabricated by other suitable means, either to a custom shape for a particular user, or to a generic shape. FIG. 11C shows an example of the tube web insert 1110 in place on the brace 120, where a reinforcement strip 1126 has been placed over the cut 1124 to refasten the ends of the cut 1124 together. Equivalent means may be used to achieve the same objective of refastening the ends of the cut 1124, including, but not limited to, fabric hook and loop fasteners, such as Velcro™, heat welding, mechanical fasteners, glues, epoxies, hot melt glues, contact cement, heat and/or catalyst activated adhesive films.

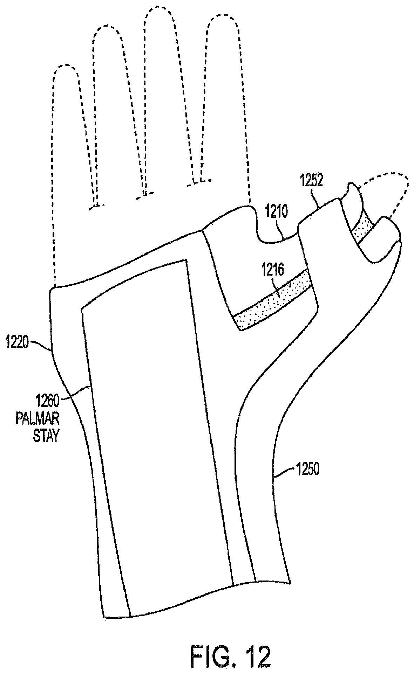

FIG. 12 shows an example of a thumb insert 1210 installed on a brace 120, and a thumb stay (splint) 1250 attached to a brace 1220 to immobilize a thumb. The brace 1220 may be substantially as described earlier, i.e., the brace 120 with the thumbhole 130 as shown in FIG. 1. Therefore, the insert may be partially outside the brace, especially in the web space area, and partially in direct contact with the thumb. Alternatively, the brace 1220 may be modified from brace 120 to expose a larger portion of the web space. Therefore, the thumb insert 1210 may be entirely in contact with the skin from the web space area of the hand to a substantial length along the side of the thumb facing the web space and a portion of the thumb. Attached to the thumb insert 1210 may be an elastic strip 1216 that may be used to attach to a part of the brace 1220 surrounding the outer side of the thumb, i.e., the side of the thumb that includes the thumb nail and faces away from the web space. The elastic strip 1216 may be sewn to the brace 1220, or alternatively attached by any of the attachment methods disclosed above, and their equivalents. The purpose of the elastic strip 1216, in addition to enabling a customized attachment of the thumb insert 1210 to the brace 1220 for comfort, is to provide a degree of flexibility and air flow breathability. The thumb insert is preferably inserted under a loop portion 1252 of the thumb stay 1250.

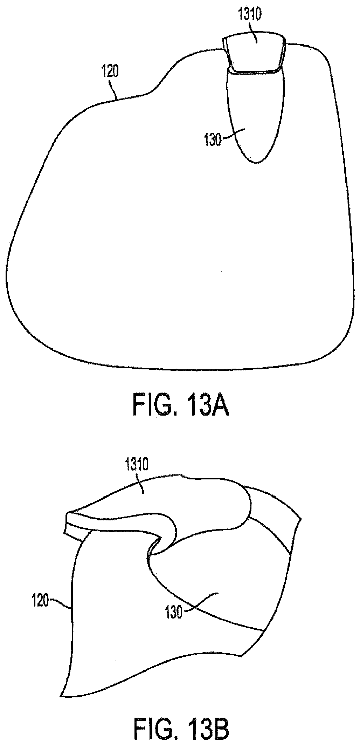

FIG 13A shows an example of a foam tape insert 1310 attached to the brace 120 in the web space area of the loop that forms the thumbhole 130. The foam tape insert 1310 may preferably have an attachment means, such as a laminated adhesive layer on one surface, as described above, or equivalents. FIG. 13B shows a detail view illustrating an example of the foam tape insert 1310 attached to the brace using a self-adhering layer of adhesive. The foam tape insert 1310 may be cut to a required size and applied to the brace 120 at a location described above, or at any other location as required.

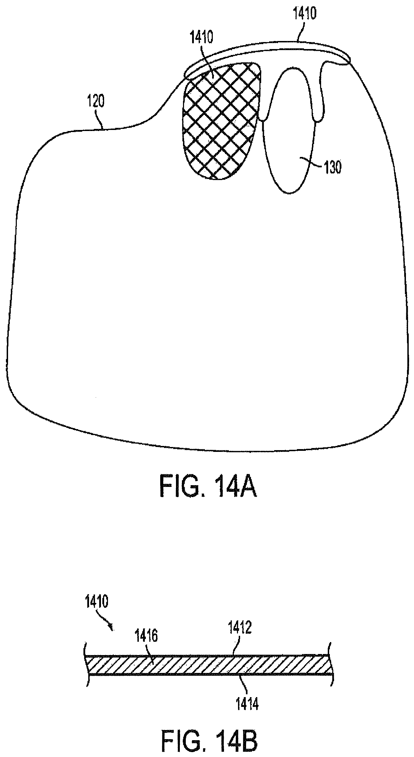

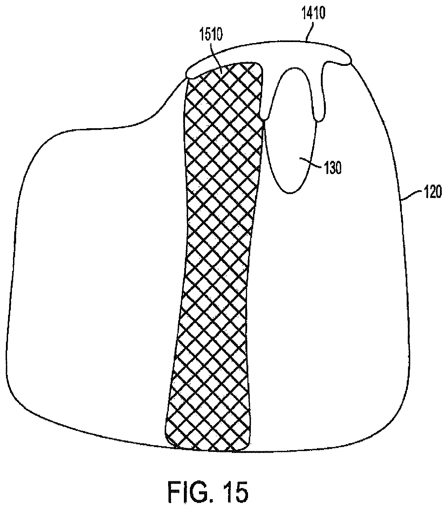

FIG. 14A shows an example of a spacer pad insert 1410 attached to one of various interior surface locations of the brace 120. For reference, a long saddle insert 410 is also shown attached to the brace 120. FIG. 14B shows the spacer pad 1410 in cross-section. In one embodiment, the spacer pad insert 1410 comprises 3D knit materials, comprising two knit faces, i.e., a first knit face 1412 and a second knit face 1414 joined by a spacer yarn 1416. The three materials may be knit in a single process. The spacer yarn 1416 may provide improved comfort and coolness via cushioning and air breathability. As a further example, FIG. 15 shows a long spacer pad 1510 located along a palmar stay region of the orthotic wrist brace 120, attached using any of various adhesive means, as described above, including, at least, fabric hook and loop fasteners, such as Velcro™, a laminated contact adhesive layer, sewing, and the like.

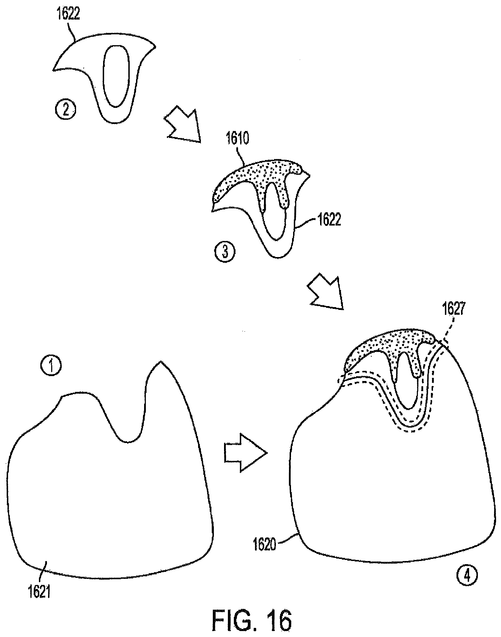

Referring to FIG. 16, there is disclosed a method of making a wrist brace 1620 which is in accordance with the present invention. The wrist brace 1620 has an integrally attached insert 1610, as shown in FIG. 16. A main body 1621 of the brace 1620 is fabricated (Step 1) to receive a second component 1622. Second component 1622 manufactured in Step 2, surrounds the base of the thumb and is shaped to receive an insert 1610 of particular type. Various types of inserts mentioned above include a donut, saddle, long saddle, and the like. Insert 1610 may be attached to second component 1622 (Step 3). Finally, the second component 1622, with insert 1610 already attached, is bonded (Step 4) to main body 1621 along a bonding interface 1627 by any of numerous attachment/bonding methods described above to form the complete wrist brace 1620.

In another embodiment, a method of making a wrist brace with an integrally attached spacer pad 1410 includes making a wrist brace 1620 and attaching the spacer pad 1410 to an interior surface of the brace 1620 at a defined location. The spacer pad 1410 may be cut or shaped as required and attached by any of the attachment means discussed above.

Various modifications to the wrist brace disclosed in FIG. 16 will be readily apparent to those skilled in the art. Thus, the claims are not intended to be limited to the embodiment of FIG. 16, but are to be accorded the full scope of the appended claims.

## Claims

1. A wrist brace comprising: a flexible body (1621) configured to provide support to the user's wrist, a component (1622) attached to the flexible body, the component having a thumbhole, the wrist brace being **characterised in** the component further comprising an insert (1610), the insert being arranged to cushion the user's web space when the user's thumb is extending through the thumbhole.

2. The wrist brace of claim 1 wherein the insert (1610) is formed with the component (1622) separately from the body.

3. The wrist brace of claim 1 wherein the insert (1610) extends over the user's palmer crease when the wrist brace is worn by the user.

4. The wrist brace of claim 1 wherein the component (1622) comprises an interior surface and an exterior surface, the insert (1610) being formed along at least one of the interior surface and the exterior surface.

5. The wrist brace of claim 1, wherein the body (1621) further comprises a palmar stay (1260).

6. The wrist brace of any proceeding claim wherein the body (1621) comprises a first material and the insert (1610) comprises a second material softer than the first material.

## Patentansprüche

1. Eine Handgelenkorthese, die Folgendes umfasst: ein flexibles Hauptteil (1621), das so konfiguriert ist, dass es das Handgelenk des Verwenders stützt, ein Element (1622), das am flexiblen Hauptteil angebracht ist, wobei das Element ein Daumenloch aufweist, wobei die Handgelenksorthese **dadurch gekennzeichnet ist, dass** sich im Element zudem ein Einsatz (1610) befindet, wobei der Einsatz so angeordnet ist, dass er die Hautfalte zwischen Daumen und Zeigefinder des Verwenders polstert, wenn der Daumen des Verwenders durch das Daumenloch herausragt.

2. Die Handgelenkorthese entsprechend Anspruch 1, wobei der Einsatz (1610) gebildet wird, indem das Element (1622) separat vom Hauptkörper ist.

3. Die Handgelenkorthese entsprechend Anspruch 1, wobei der Einsatz (1610) sich über die Handflächenfalte des Verwenders erstreckt, wenn die Handgelenkorthese vom Verwender getragen wird.

4. Die Handgelenkorthese entsprechend Anspruch 1, wobei das Element (1622) eine Innenfläche und eine Außenfläche umfasst, wobei der Einsatz (1610) entlang von zumindest entweder der Innenfläche oder der Außenfläche gebildet wird.

5. Die Handgelenkorthese entsprechend Anspruch 1, wobei das Hauptteil (1621) zudem eine Handflächenstrebe (1260) umfasst.

6. Die Handgelenkorthese entsprechend einem der vorhergehenden Ansprüche, wobei das Hauptteil (1621) ein erstes Material umfasst und der Einsatz (1610) ein zweites Material umfasst, das weicher als das erste Material ist.

## Revendications

1. Une atèle de poignet comprenant : un corps flexible (1621) configuré pour soutenir le poignet de l'utilisateur, un composant (1622) attaché au corps flexible, ce composant étant doté d'une ouverture permettant de passer le pouce, l'atèle de poignet étant **caractérisée en ce que** ledit composant comprend en outre un coussinet (1610) disposé pour rembourrer l'espace de l'utilisateur lorsque celui-ci fait passer son pouce par l'ouverture destinée à cet effet.

2. L'atèle de poignet de la revendication 1 dans laquelle le coussinet (1610) est formé avec le composant (1622) séparément du corps.

3. L'atèle de poignet de la revendication 1 dans laquelle le coussinet (1610) s'étend sur le pli de la paume de l'utilisateur lorsque celui-ci porte l'atèle de poignet.

4. L'atèle de poignet de la revendication 1 dans laquelle le composant (1622) comprend une surface interne et une surface externe, le coussinet (1610) étant formé le long d'au moins une des surfaces interne et externe.

5. L'atèle de poignet de la revendication 1, dans laquelle le corps (1621) comprend en outre un support de paume (1260).

6. L'atèle de poignet de l'une quelconque des revendications précédentes dans laquelle le corps (1621) comprend un premier matériau et le coussinet (1610) comprend un second matériau plus souple que le premier matériau.
